# FASCICULE DE BREVET EUROPEEN

(11) **EP 2 081 525 B1**
(45) Date de publication et mention de la délivrance du brevet: **16.06.2010**
(21) Numéro de dépôt: 07846711.5
(22) Date de dépôt: 21.11.2007
(51) Int. Cl.: A61F 5/56

(54) **DISPOSITIF DESTINE A PROVOQUER UN DEPLACEMENT DE LA MANDIBULE PAR RAPPORT AU MAXILLAIRE**
VORRICHTUNG ZUR ERZEUGUNG EINER VERSCHIEBUNG DES UNTERKIEFERS RELATIV ZUM OBERKIEFER
DEVICE FOR GENERATING A DISPLACEMENT OF THE MANDIBULA RELATIVE TO THE MAXILLA

(30) Priorité: 23.11.2006 FR 0610284
(43) Date de publication de la demande: 29.07.2009
(73) Titulaire: Bonnaure, Pierre, 35700 Rennes (FR)
(72) Inventeur: Bonnaure, Pierre, 35700 Rennes (FR)
(74) Mandataire: Maillet, Alain
(86) Numéro de dépôt international: PCT/EP2007/010077
(87) Numéro de publication internationale: WO 2008/061725

(56) Documents cités:
- WO-A-03/034957
- DE-A1- 10 331 531
- US-A- 6 012 920
- US-B1- 6 526 982

## Description

La présente invention concerne un dispositif destiné à provoquer un déplacement de la mandibule par rapport au maxillaire d'un utilisateur. Le document WO 03/034 957 A représente l'état de la technique le plus proche et détermine le préambule de la revendication 1.

Les dispositifs connus destinés à provoquer un déplacement de la mandibule sont des dispositifs permettant de provoquer un avancement de la mandibule par rapport au maxillaire, et sont autrement nommés orthèses. De tels dispositifs sont utilisés pour le traitement de troubles respiratoires obstructifs du sommeil, tels que les syndromes de haute résistance des voies aériennes supérieures, les ronflements, les apnées et les hyponées.

Les troubles respiratoires obstructifs du sommeil sont dus à une mauvaise ventilation du sujet au cours de son sommeil par obstruction de ses voies respiratoires. Les voies respiratoires, en particulier le pharynx, sont obstruées, par exemple, dans le cas d'une luette trop volumineuse, d'un voile du palais trop épais, d'une position de la mandibule en arrière du maxillaire ou d'une mauvaise position de la base de la langue.

Les conséquences des troubles respiratoires obstructifs du sommeil sont nombreuses comme, par exemple, une somnolence diurne, des difficultés de concentration, une dépression ou un grand nombre de problèmes psychologiques.

Chez l'enfant, les troubles respiratoires obstructifs du sommeil sont également courants. Ils ont pour conséquences différents problèmes, tels que des problèmes de croissance, des problèmes d'hyperactivité, des problèmes de concentration ou encore une absence d'élargissement de l'arcade palatine. Une des causes de ces troubles est une mauvaise croissance de la mandibule dans le sens postéro antérieur avec pour conséquence une obstruction des voies respiratoires. La prise en charge d'un problème de croissance de la mandibule chez l'enfant permet d'éviter les troubles obstructifs du sommeil à l'âge adulte.

La médecine dispose d'un traitement efficace mais à la fois lourd et coûteux : la ventilation par pression positive continue (VPPC). De manière à être correctement ventilé, le patient doit porter, pendant la nuit, un masque lui insufflant de l'air par le nez. Malheureusement, certains patients ne supportent pas le bruit occasionné par la machine.

Il est également possible d'avoir recours à la chirurgie, par exemple, pour couper le voile du palais lorsqu'il est trop épais ou pour avancer le menton.

Une autre solution moins onéreuse, et comportant moins de risques, consiste en l'utilisation d'orthèse d'avancée mandibulaire. Le principe consiste à propulser en avant et de quelques millimètres, la mandibule par rapport à sa position d'origine. Cet avancement permet le dégagement du pharynx et permet ainsi une ventilation correcte du sujet.

L'orthèse d'avancement mandibulaire la plus connue est sans doute la bielle de Herbst. Pour la mise en place de cette orthèse, les rangées de dents du haut et du bas sont chacune recouvertes d'une gouttière rigide réalisée en résine acrylique thermoformée et bien connue de l'homme du métier. Deux bielles métalliques sont disposées respectivement de chaque côté des arcades dentaires supérieure et inférieure, sur leur face externe, de façon à relier la rangée de dents du haut et la rangée de dents du bas. Les deux bielles sont attachées à la gouttière recouvrant la rangée de dents du haut par des moyens d'ancrage disposés au niveau des deux dernières molaires, respectivement. Les deux bielles sont attachées à la gouttière recouvrant la rangée de dents du bas par des moyens d'ancrage disposés au niveau des deux canines, respectivement.

On connaît encore des orthèses composées de deux gouttières thermoformées recouvrant la rangée de dents du haut et la rangée de dents du bas, respectivement, et comprenant de plus, deux élastiques fixés dans des crochets reliant latéralement les deux gouttières. La mâchoire inférieure est maintenue ainsi en position avancée.

Ces dispositifs d'avancement mandibulaire présentent l'inconvénient d'être parfois très encombrants. Pour certains, tels que ceux utilisant une bielle de Herbst, la rigidité peut occasionner une gêne pour l'utilisateur. De plus, aucun dispositif connu ne présente de possibilité de réglage fin et ces dispositifs se contentent donc de pousser la mandibule dans une direction postéro antérieure par rapport au maxillaire supérieur et ne permettent pas de tracter la mandibule également dans une direction à la fois parallèle au plan formé par le maxillaire supérieur et perpendiculaire à la direction postéro antérieure.

La présente invention concerne un dispositif d'avancement mandibulaire qui ne présente pas les inconvénients précédemment cités. En particulier, l'invention a pour but de proposer un dispositif qui ne soit ni gênant pour l'utilisateur, ni encombrant, et qui permette un déplacement de la mandibule par rapport au maxillaire supérieur dans une direction postéro antérieure et/ou une direction transversale par rapport au plan du maxillaire et/ou perpendiculaire par rapport au plan du maxillaire.

Dans la présente invention, une direction dite transversale par rapport au plan du maxillaire est une direction à la fois parallèle au plan formé par le maxillaire supérieur et perpendiculaire à la direction postéro antérieure.

A cet effet, un dispositif selon l'invention, destiné à provoquer un déplacement de la mandibule par rapport au maxillaire supérieur lorsqu'il est porté par un utilisateur, est définie dans la revendication 1.

Le dispositif selon l'invention présente ainsi l'avantage de comporter un moyen de réglage du déplacement de la mandibule par rapport au maxillaire supérieur et ceci à la fois dans une direction postéro antérieure par rapport au plan du maxillaire supérieur et une direction dite transversale par rapport à ce même plan. En effet, chaque moyen de déplacement mandibulaire comporte son propre moyen de réglage du déplacement de la mandibule ce qui permet de régler le déplacement de la mandibule dans le sens postéro antérieur. Si le dispositif comporte au moins deux moyens de déplacement mandibulaire, des réglages différents de chaque moyen de réglage provoqueront un déplacement de la mandibule dans le sens postéro antérieur et dans le sens transversal par rapport au plan du maxillaire supérieur.

Le moyen de réglage du déplacement mandibulaire présente également l'avantage de pouvoir régler ce déplacement de la mandibule par rapport au maxillaire de façon progressive, de période en période.

De plus, la structure en deux parties de chaque moyen de déplacement mandibulaire, c'est-à-dire, une partie inférieure et une partie supérieure pouvant être reliées entre elles par un moyen de liaison, présente l'avantage de faciliter l'utilisation du dispositif selon l'invention, les deux parties pouvant être reliées avant la pose en bouche ou après.

Selon un mode de réalisation préféré de l'invention, ledit moyen de liaison et ledit moyen de réglage sont un seul et même moyen.

Selon un mode de réalisation préféré de l'invention, ledit moyen de réglage est composé d'un tube taraudé que comporte ladite partie inférieure et d'un filetage que comporte ladite partie supérieure, ou vice versa, ledit filetage étant prévu pour être vissé dans ledit tube taraudé.

Ainsi, la liaison entre la partie inférieure et la partie supérieure ainsi que le réglage du déplacement de la mandibule par rapport au maxillaire sont réalisés par vissage du filetage dans le tube taraudé.

Selon un autre mode de réalisation, ledit moyen de réglage est composé d'au moins un cran que comporte ladite partie inférieure et d'au moins une encoche que comporte ladite partie supérieure, ou vice versa, ledit cran étant prévu pour s'emboîter dans ladite encoche.

Ainsi, la liaison entre la partie inférieure et la partie supérieure ainsi que le réglage du déplacement de la mandibule par rapport au maxillaire sont réalisés par crantage, c'est-à-dire par progression d'un cran d'encoche en encoche.

De façon avantageuse, ledit dispositif comporte un moyen de blocage dudit moyen de réglage.

Dans un mode de réalisation de l'invention, ledit moyen de blocage est un boulon vissé sur ledit filetage.

Une fois le déplacement de la mandibule par rapport au maxillaire réglé par le moyen de réglage, il peut être avantageux de bloquer ce réglage. Le moyen de blocage permet de bloquer la position de la mandibule par rapport au maxillaire. Par exemple, lorsque le moyen de réglage est composé par un filetage vissé dans un tube taraudé, il permet d'éviter la rotation du tube taraudé autour du filetage, autrement dit, que le filetage ne se visse ou se dévisse plus encore.

Selon une caractéristique de l'invention, la partie inférieure dudit moyen de déplacement mandibulaire comporte une tige souple. Cette caractéristique permet une grande liberté de mouvement une fois le dispositif porté par l'utilisateur et donc un grand confort.

Selon un mode de réalisation de l'invention, le moyen de fixation dudit moyen de déplacement mandibulaire au maxillaire supérieur est disposé au niveau de la canine et/ou des prémolaires de ladite arcade dentaire supérieure et ledit moyen de fixation dudit moyen de déplacement mandibulaire sur la mandibule est disposé au niveau des molaires de ladite arcade dentaire inférieure lorsque ledit dispositif est porté par un utilisateur.

Une fois le dispositif porté par l'utilisateur, le moyen de déplacement mandibulaire n'est donc pas perpendiculaire au plan formé par le maxillaire. Ceci permet le déplacement de la mandibule dans le sens postéro antérieur par rapport au maxillaire.

Selon un mode de réalisation de l'invention, ledit moyen de fixation dudit moyen de déplacement mandibulaire sur le maxillaire supérieur est composé d'un tube destiné à loger une extrémité de ladite partie supérieure dudit moyen de déplacement mandibulaire, ledit tube étant prévu pour être fixé sur ladite arcade dentaire supérieure dudit maxillaire supérieur directement, ou par l'intermédiaire d'un moyen de liaison.

Selon un autre mode de réalisation de l'invention, ledit moyen de fixation dudit moyen de déplacement mandibulaire sur le maxillaire supérieur est composé d'un anneau que comporte ladite partie supérieure dudit moyen de déplacement mandibulaire et d'un crochet fixé audit maxillaire supérieur directement ou par l'intermédiaire d'un moyen de liaison, ledit anneau étant prévu pour être crocheté sur ledit crochet.

Selon un mode de réalisation de l'invention, ledit moyen de fixation dudit moyen de déplacement mandibulaire sur la mandibule est composé d'un anneau que comporte ladite partie inférieure dudit moyen de déplacement mandibulaire et d'un crochet fixé à ladite mandibule directement ou par l'intermédiaire d'un moyen de liaison, ledit anneau étant prévu pour être crocheté sur ledit crochet.

Dans un mode de réalisation préféré de l'invention, ledit moyen de fixation dudit moyen de déplacement mandibulaire sur la mandibule est composé d'un moyen de liaison à ladite mandibule dans laquelle une extrémité de ladite partie inférieure dudit moyen de déplacement mandibulaire est coulée.

De façon avantageuse, ledit moyen de liaison est choisi parmi une gouttière thermoformée, un implant dentaire, une couronne dentaire, une bague d'appareil d'orthodontie, au moins un micro-implant ou au moins une mini-vis vissés dans la gencive ou sous la gencive.

La gouttière thermoformée, l'implant dentaire, la couronne dentaire ou la bague d'appareil d'orthodontie, sont disposés au niveau d'une arcade dentaire. Le ou les micro-implant(s) ou la ou les mini-vis sont vissés dans l'os au travers de la gencive ou sous la gencive. Ces derniers peuvent être, par exemple, utilisés chez un utilisateur d'un dispositif selon l'invention dont le maxillaire supérieur et/ou la mandibule ne comporterait pas d'arcade dentaire supérieure et/ou inférieure, respectivement.

Dans un autre mode de réalisation préféré de l'invention, ledit dispositif comporte à la fois une gouttière thermoformée destinée à recouvrir au moins une partie de ladite arcade dentaire inférieure et une gouttière thermoformée destinée à recouvrir au moins une partie de ladite arcade dentaire supérieure.

Selon une caractéristique de l'invention, la partie supérieure dudit moyen de déplacement mandibulaire comporte une tige en forme de U dont une branche est destinée à être fixée sur ledit maxillaire supérieur par ledit moyen de fixation dudit moyen de déplacement mandibulaire audit maxillaire supérieur.

Le dispositif selon l'invention peut comporter, en outre, au moins une cale prévue pour être fixée à une desdites arcades dentaires supérieure ou inférieure directement ou par un moyen de liaison. Ladite cale permet que la bouche de l'utilisateur soit maintenue plus ou moins ouverte lorsque le dispositif est porté par l'utilisateur.

Ledit moyen de liaison est choisi, par exemple, parmi une gouttière thermoformée, un implant dentaire, une couronne dentaire ou une bague d'appareil d'orthodontie.

Le dispositif selon l'invention peut comporter, de plus, au moins un élastique prévu pour être fixé audit maxillaire supérieur et à ladite mandibule par deux moyens d'accrochage, respectivement, fixés sur ledit maxillaire supérieur et sur ladite mandibule directement ou par l'intermédiaire de deux moyens de liaison, respectivement, tels que lesdits moyens de liaison décris ci-dessus.

Ledit élastique permet que la bouche de l'utilisateur soit maintenue plus ou moins fermée lorsque le dispositif est porté par l'utilisateur.

Ladite cale et ledit élastique permettent ainsi de déplacer la mandibule par rapport au maxillaire dans une direction perpendiculaire au plan du maxillaire.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
La Fig. 1 représente un dispositif destiné à provoquer un déplacement de la mandibule par rapport au maxillaire supérieur d'un utilisateur, selon un mode préféré de réalisation de l'invention,
La Fig. 2 représente un dispositif destiné à provoquer un déplacement de la mandibule par rapport au maxillaire supérieur d'un utilisateur, selon un autre mode de réalisation de l'invention et,
Les Figs 3a et 3b illustrent un moyen de réglage du déplacement mandibulaire selon un autre mode de réalisation de l'invention.

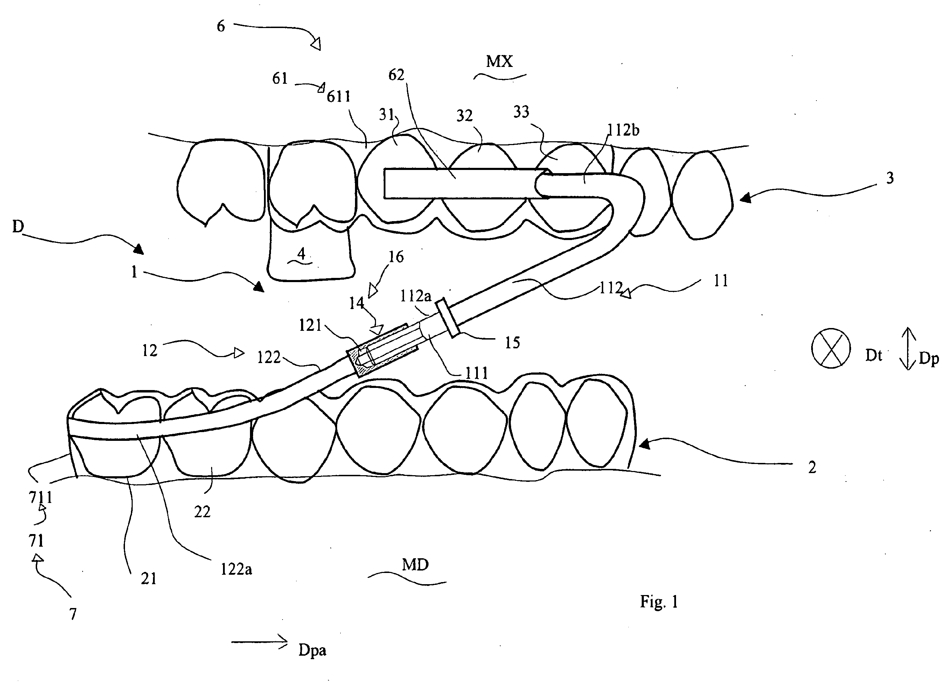

Sur la Fig. 1 est représenté le maxillaire supérieur MX comportant l'arcade dentaire supérieure 3 ainsi que la mandibule MD comportant l'arcade dentaire inférieure 2 d'un utilisateur du dispositif D selon l'invention.

Le dispositif D est destiné à permettre le déplacement de la mandibule MD par rapport au maxillaire supérieur MX dans une direction postéro antérieure Dpa et/ou une direction transversale Dt par rapport au plan du maxillaire MX et/ou perpendiculaire Dp par rapport au plan du maxillaire supérieur MX.

Le dispositif D comporte un moyen de déplacement mandibulaire 1, lequel comporte une partie inférieure 12 et une partie supérieure 11 qui sont reliées entre elles par l'intermédiaire d'un moyen de liaison 14.

Le moyen de liaison 14 est composé d'un filetage 111 que comporte la partie supérieure 11 et d'un tube taraudé 121 que comporte la partie inférieure 12. Le filetage 111 est vissé dans le tube taraudé 121 de manière à relier la partie supérieure 11 à la partie inférieure 12.

La partie supérieure 11 comporte une tige 112 en forme de U. Une branche 112a de ladite tige 112 comporte le filetage 111. Une autre branche 112b de la tige 112 est logée à l'intérieur d'un tube 62, lequel est fixé sur l'arcade supérieure 3 par l'intermédiaire d'un moyen de liaison 61. Le moyen de liaison 61 est, par exemple, une gouttière thermoformée 611, bien connue de l'homme du métier, et qui recouvre une partie de l'arcade dentaire 3 comprenant au moins les prémolaires 31, 32 et la canine 33. Le tube 62 et le moyen de liaison 61 forment un moyen de fixation 6 du moyen de déplacement mandibulaire 1 au maxillaire supérieur MX. Le moyen de fixation 6 est disposé au niveau des prémolaires 31, 32 et de la canine 33.

La gouttière thermoformée 611 peut être amovible ou scellée, par exemple collée, sur une partie ou sur la totalité de l'arcade dentaire 3.

La partie inférieure 12 comporte une tige 122, avantageusement réalisée en matériau souple de manière à permettre un mouvement volontaire de la mandibule MD.

La partie inférieure 12 du moyen de déplacement mandibulaire 1 est fixée à la mandibule MD par un moyen de fixation 7.

Le moyen de fixation 7 est constitué par un moyen de liaison 71 du dispositif 1 à la mandibule MD, dans lequel une extrémité 122a de la partie inférieure 12 est coulée. Ce moyen de liaison 71 est une gouttière thermoformée 711, laquelle recouvre la totalité ou une partie de l'arcade dentaire inférieure 2 et peut être amovible.

L'extrémité 122a de la partie inférieure 12 est coulée au niveau des molaires 21, 22. Le moyen de déplacement mandibulaire 1 n'est donc pas perpendiculaire au plan formé par le maxillaire MX.

La gouttière thermoformée 711 recouvre, par exemple, la totalité de l'arcade dentaire 2 et est amovible.

Le moyen de liaison 14, dans ce mode de réalisation, est également un moyen de réglage 16 du déplacement de la mandibule MD par rapport au maxillaire MX. En effet, le vissage du filetage 111 dans le tube taraudé 121 provoque le déplacement de la mandibule MD par rapport au maxillaire MX dans une direction postéro antérieure Dpa. La progression du filetage 111 dans le tube taraudé 121 peut ainsi être réalisée de période en période, par exemple, de jour en jour.

Le dispositif D comporte, dans un mode de réalisation préféré de l'invention, un second moyen de déplacement mandibulaire (non représenté) prévu pour être disposé symétriquement par rapport à un axe central longitudinal parallèle au plan du maxillaire supérieur MX, par exemple, de chaque côté des arcades dentaires 2 et 3.

Il est alors possible de régler le déplacement de la mandibule MD par rapport au maxillaire supérieur MX, à la fois dans une direction transversale Dt par rapport au plan du maxillaire supérieur MX et dans une direction postéro antérieure Dpa par rapport au maxillaire MX. En effet, les vissages des filetages 111 dans les tubes taraudés 121 des deux moyens de déplacement 1, respectivement, du dispositif D, peuvent être différents ce qui provoque un déplacement de la mandibule dans une direction transversale Dt par rapport au plan du maxillaire MX en plus du déplacement dans la direction postéro antérieure Dpa.

Le dispositif D, comme illustré sur la Fig. 1, peut encore comporter un moyen de blocage 15 du moyen de réglage 16. Ledit moyen de blocage 15 peut, par exemple, comporter un écrou vissé sur le filetage 111, de manière à empêcher toute rotation du tube taraudé 121 autour du filetage 111.

Le dispositif D peut également comporter une cale 4 fixée, par exemple collée, à l'arcade dentaire 3 par l'intermédiaire du moyen de liaison 61, par exemple, de la gouttière thermoformée. La cale 4 permet d'éviter que les arcades dentaires supérieure et inférieure 3 et 2, respectivement, ne se rencontrent. Ainsi, la cale 4 permet un déplacement de la mandibule MD par rapport au maxillaire MX dans une direction perpendiculaire Dp par rapport au plan du maxillaire MX.

Dans une variante de l'invention (non représentée), le dispositif peut comporter un élastique prévu pour être fixé au maxillaire supérieur MX et à la mandibule MD par l'intermédiaire de deux moyens de liaison, respectivement, tels que les moyens de liaison 61 et 71 décrits ci-dessus. L'élastique permet de maintenir les arcades dentaires supérieure et inférieure 3 et 2, respectivement, l'une contre l'autre. L'élastique permet également un déplacement de la mandibule MD par rapport au maxillaire MX dans une direction perpendiculaire Dp par rapport au plan du maxillaire MX.

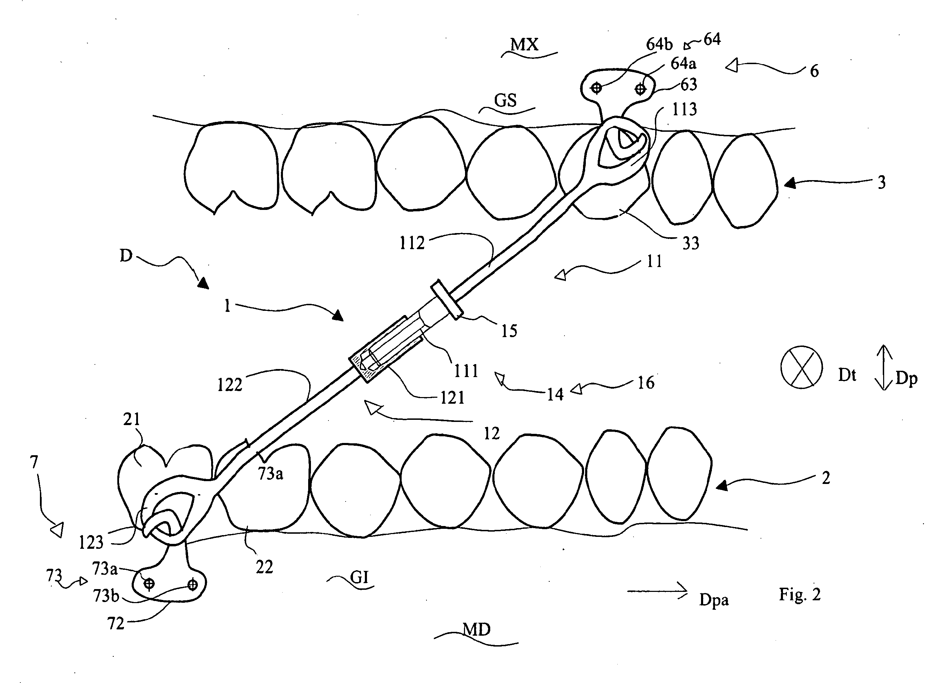

Sur la Fig. 2, est représenté un autre mode de réalisation du dispositif D selon l'invention.

Le dispositif D comprend un moyen de déplacement mandibulaire 1 comportant une partie supérieure 11 et une partie inférieure 12.

La partie inférieure 12 comporte une tige souple 122 dont une extrémité est conformée en anneau 123. De même, la partie supérieure 11 comporte une tige 112 dont une extrémité est conformée en anneau 113.

Le dispositif D comporte un moyen de liaison 14 qui est également un moyen de réglage 16 identique à celui du mode de réalisation décrit ci-dessus.

La partie inférieure 12 du moyen de déplacement mandibulaire 1 est fixée à la mandibule MD par un moyen de fixation 7. Le moyen de fixation 7 est composé de l'anneau 123 de la partie inférieure 12 et d'un crochet 72 fixé à la mandibule par l'intermédiaire d'un moyen de liaison 73. Le moyen de liaison 73 est composé d'au moins un micro-implant, par exemple, de deux micro-implants 73a et 73b, qui sont vissés dans ou sous la gencive inférieure GI au-dessous de la molaire 21 ou entre les molaires 21 et 22. Le moyen de fixation 7 est donc disposé au niveau des molaires de l'utilisateur.

La partie supérieure 11 du moyen de déplacement mandibulaire 1 comporte une tige 112 dont une extrémité est conformée en anneau 123.

La partie supérieure 11 est fixée au maxillaire MX par un moyen de fixation 6. Le moyen de fixation 6 est composé de l'anneau 113 que comporte la partie supérieure 11 et d'un crochet 63 fixé au maxillaire MX par l'intermédiaire d'un moyen de liaison 64. Le moyen de liaison 64 est composé d'au moins un micro-implant, par exemple, de deux micro-implants 64a et 64b qui sont vissés dans ou sous la gencive supérieure GS au-dessus de la canine 33.

Le moyen de fixation 6 est donc disposé au niveau de la canine de l'utilisateur.

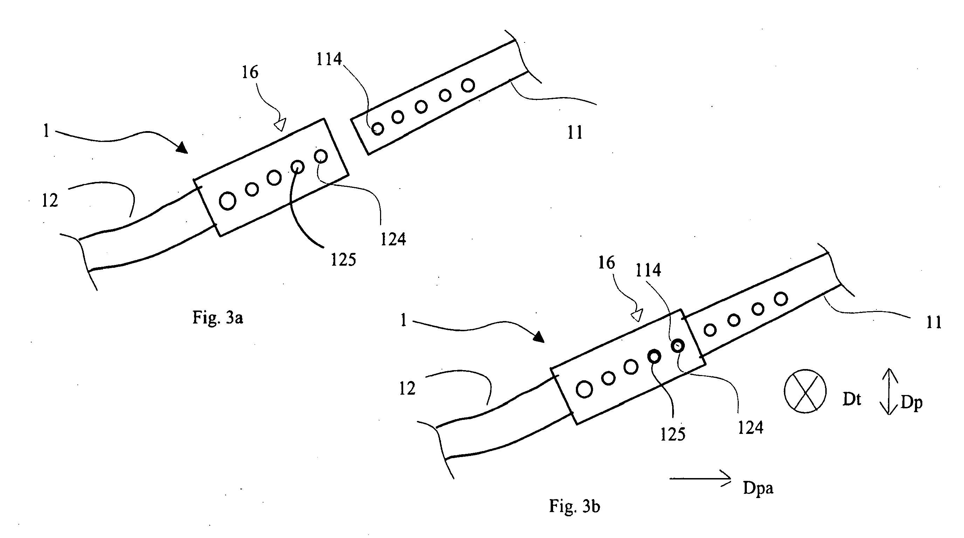

Dans une autre variante de l'invention illustrée sur les Figs. 3a et 3b, la partie supérieure 11 du moyen de déplacement mandibulaire 1 comporte des crans tels que le cran 114 et la partie inférieure 12 comporte des encoches telles que les encoches 124 (Fig. 3a). Les crans, tels que le cran 114 et les encoches, telles que l'encoche 124, constituent le moyen de réglage 16 du déplacement de la mandibule par rapport au maxillaire. L'homme du métier comprendra que les crans, tels que le cran 114, peuvent, à l'inverse, se trouver dans la partie inférieure 12 si les encoches, telles que l'encoche 124, se trouvent dans la partie supérieure 11. Les crans, tels que le cran 114, sont prévus pour s'emboîter dans les encoches, telles que l'encoche 124.

Dans cette variante de l'invention, le moyen de réglage 16 et le moyen de liaison 14 de la partie inférieure 12 et de la partie supérieure 11 sont un seul et même moyen. En effet, lorsque le cran 114 est emboîté dans l'encoche 124, la liaison entre la partie inférieure 12 et la partie supérieure 11 est établie.

Dans cette variante de l'invention, le réglage du déplacement de la mandibule par rapport au maxillaire dans la direction postéro antérieure Dpa se fait par crantage, c'est-à-dire par progression du cran 114 d'une encoche 124 à une autre encoche 125 (Fig. 3b).

De même que dans le premier mode de réalisation décrit ci-dessus, le dispositif D peut comporter un second moyen de déplacement mandibulaire (non représenté) comportant le moyen de réglage 16 illustré sur les Figs. 3a et 3b. Il est alors possible de régler le déplacement de la mandibule MD par rapport au maxillaire supérieur MX, à la fois dans une direction transversale Dt par rapport au plan du maxillaire supérieur MX et dans une direction postéro antérieure Dpa par rapport au maxillaire MX. En effet, la progression des crans, tels que le cran 114, dans les encoches, telles que les encoches 124 ou 125, des deux moyens de déplacement 1 peut être différente, ce qui provoque un déplacement de la mandibule dans une direction transversale Dt par rapport au plan du maxillaire MX en plus du déplacement dans la direction postéro antérieure Dpa.

Le dispositif selon l'invention permet ainsi de régler le déplacement de la mandibule par rapport au maxillaire d'un utilisateur dans plusieurs directions. Les applications d'un tel dispositif selon l'invention sont nombreuses. Par exemple, le dispositif peut être utilisé chez l'adulte pour lutter contre les troubles obstructifs du sommeil, tels que le syndrome de haute résistance des voies aériennes supérieures, les apnées et les ronflements. Chez l'enfant, le dispositif peut être utilisé de façon à favoriser la croissance mandibulaire. En particulier, le dispositif peut être utilisé pour favoriser la croissance mandibulaire simultanément au traitement de tout autre problème d'orthodontie ou de mâchoire, par exemple, conjointement à une disjonction palatine.

## Revendications

1. Dispositif (D) destiné à provoquer un déplacement de la mandibule (MD) par rapport au maxillaire supérieur (MX), lorsqu'il est porté par un utilisateur, ladite mandibule (MD) et ledit maxillaire (MX) pouvant comporter une arcade dentaire inférieure (2) et une arcade dentaire supérieure (3), respectivement, ledit dispositif (D) comportant au moins un moyen de déplacement mandibulaire (1) dont une partie supérieure (11) est prévue pour être reliée audit maxillaire supérieur (MX) par l'intermédiaire d'un moyen de fixation (6) et une partie inférieure (12) est prévue pour être reliée à ladite mandibule (MD) par un moyen de fixation (7) composé d'une gouttière (711), par exemple thermoformée, lesdites parties supérieure (11) et inférieure (12) étant prévues pour être reliées l'une à l'autre par l'intermédiaire d'un moyen de liaison (14) **caractérisé en ce que** ladite partie supérieure (11) comporte une tige (112) en forme de U dont une branche (112b) est destinée à être logée à l'intérieur d'un tube (62) que comporte le moyen de fixation (6) du dispositif (D) sur ledit maxillaire (MX) et **en ce que** ladite partie inférieure (12) comporte une tige (122) souple dont une extrémité (122a) est coulée dans le moyen de fixation (7) dudit dispositif (D) à la mandibule (MD).

2. Dispositif (D) selon la revendication 1, **caractérisé en ce que** ledit moyen de fixation (6) comporte une gouttière (611), par exemple thermoformée, un implant dentaire, une couronne dentaire, une bague d'appareil d'orthodontie, au moins un micro-implant (64a, 64b) ou au moins une mini-vis.

3. Dispositif (D) selon l'une des revendications précédentes, **caractérisé en ce que** ledit moyen de fixation (6) dudit moyen de déplacement mandibulaire (1) au maxillaire supérieur (MX) est disposé au niveau de la canine (33) et/ou des prémolaires (31, 32) de l'arcade dentaire supérieure (3) et ledit moyen de fixation (7) dudit moyen de déplacement mandibulaire (1) sur la mandibule (MD) est disposé au niveau d'une ou des molaires (21, 22) de l'arcade dentaire inférieure (2) lorsque ledit dispositif (D) est porté par l'utilisateur.

4. Dispositif (D) selon l'une des revendications précédentes, **caractérisé en ce que** ledit moyen de liaison (14) comporte un moyen de réglage (16) du déplacement de la mandibule (MD) par rapport au maxillaire supérieur (MX), composé d'un filetage (111) que comporte ladite partie supérieure (11) et d'un tube taraudé (121) que comporte la partie inférieure (12), ou vice versa, ledit filetage (111) étant vissé sur ledit tube taraudé (121).

5. Dispositif (D) selon l'une des revendications précédentes, **caractérisé en ce qu'**il comporte, en outre, au moins une cale (4) prévue pour être fixée à une desdites arcades dentaires supérieure ou inférieure (3, 2) directement ou par l'intermédiaire d'un moyen de liaison (61).

6. Dispositif (D) selon l'une des revendications précédentes, **caractérisé en ce qu'**il comporte, en outre, au moins un élastique prévu pour être fixé audit maxillaire supérieur (MX) et à ladite mandibule (MD) par deux moyens d'accrochage, respectivement, fixés sur ledit maxillaire supérieur (MX) et sur ladite mandibule (MD) directement ou sur lesdits moyens de liaison (6, 7).

## Claims

1. Device (D) intended to produce displacement of the mandible (MD) relative to the upper maxilla (MX) when worn by a user, wherein the said mandible (MD) and the said maxilla may comprise a lower dental arch (2) and an upper dental arch (3), respectively, the said device (D) comprising at least one mandibular displacement means (1), an upper part (11) of which is adapted to be connected to said upper maxilla (MX) via a fixing means (6) and a lower part (12) of which is adapted to be connected to said mandible (MD) via a fixing means (7) made up of a trough (711), which may for example be thermoformed, the said upper (11) and lower (12) parts being adapted to be connected to one another by means of a connecting member (14), **characterised in that** the upper part (11) comprises a rod (112) in the shape of a U, one arm (112b) of which is intended to be accommodated inside a tube (62) provided on the means (6) for fixing the device (D) to said maxilla (MX) and **in that** said lower part (12) comprises a flexible rod (122) one end (122a) of which is cast in the means (7) for fixing said device (D) to the mandible (MD).

2. Device (D) according to claim 1, **characterised in that** the said fixing means (6) comprise a trough (611) which may for example be thermoformed, a dental implant, a dental crown, an orthodontic ring, at least one micro-implant (64a, 64b) or at least one mini-screw.

3. Device (D) according to one of the preceding claims, **characterised in that** the said means (6) for fixing the mandibular displacement means (1) to the upper maxilla (MX) are located at the level of the canine (33) and/or the premolars (31, 32) of the upper dental arch (3) and the said means (7) for fixing the mandibular displacement means (1) to the mandible (MD) are located at the level of one or more molars (21, 22) of the lower dental arch (2) when said device (D) is worn by the user.

4. Device (D) according to one of the preceding claims, **characterised in that** the said connecting means (14) comprise means (18) for adjusting the displacement of the mandible (MD) relative to the upper maxilla (MX), comprising a thread (111) provided on the upper part (11) and a tapped tube (121) provided on the lower part (12), or *vice versa*, the said thread (111) being screwed to said tapped tube (121).

5. Device (D) according to one of the preceding claims, **characterised in that** it further comprises at least one wedge (4) adapted to be fixed to one of said upper or lower dental arches (3, 2) directly or via a connecting member (61).

6. Device (D) according to one of the preceding claims, **characterised in that** it further comprises at least one elastic band adapted to be fixed to said upper maxilla (MX) and to said mandible (MD) by two attachment means, respectively, fixed to said upper maxilla (MX) and to said mandible (MD) directly or to said connecting members (6, 7).

## Patentansprüche

1. Vorrichtung (D) zur Erzeugung einer Verlagerung des Unterkiefers (MD) relativ zum Oberkiefer (MX), wenn sie von einem Benutzer getragen wird, wobei der Unterkiefer (MD) und der Oberkiefer (MX) eine untere Zahnreihe (2) bzw. eine obere Zahnreihe (3) aufweisen können, wobei die Vorrichtung (D) wenigstens ein Mittel zur Kieferverlagerung (1) aufweist, von dem ein oberer Bereich (11) dazu vorgesehen ist, mittels eines Befestigungsmittels (6) an dem Oberkiefer (MX) befestigt zu werden, und von dem ein unterer Bereich (12) dazu vorgesehen ist, mittels eines aus einer beispielsweise wärmegeformten Schiene (711) bestehenden Befestigungsmittels (7) an dem Unterkiefer (MD) befestigt zu werden, wobei der obere Bereich (11) und der untere Bereich (12) dazu vorgesehen sind, durch ein Verbindungsmittel (14) miteinander verbunden zu werden,
**dadurch gekennzeichnet, dass**
- der obere Bereich (11) einen U-förmigen Stift (112) umfasst, von dem ein Zweig (112b) dazu vorgesehen ist, im Inneren eines Rohres (62) das die Befestigungsmittel (6) der Vorrichtung (D) an dem Oberkiefer (MX) aufweisen, angeordnet zu sein und
- der untere Bereich (12) einen biegsamen Stift (122) aufweist, von dem ein Ende (112a) in dem Befestigungsmittel (7) der Vorrichtung (D) am Unterkiefer (MD) eingegossen ist.

2. Vorrichtung (D) nach Anspruch 1,
**dadurch gekennzeichnet, dass**
das Befestigungsmittel (6) eine beispielsweise wärmegeformte Schiene (611), ein Zahnimplantat, eine Zahnkrone, einen Ring eines kieferorthopädischen Geräts, wenigstens ein Mikroimplantat (641, 64b) oder zumindest eine Mikroschraube umfasst.

3. Vorrichtung (D) nach einem der vorhergehenden Ansprüche,
**dadurch gekennzeichnet, dass**
- die Befestigungsmittel (6) des Kieferverlagerungsmittels (1) am Oberkiefer (MX) auf der Höhe des Eckzahns (33) und/oder des vorderen Backenzahns (31, 32) des oberen Zahnbogens (3) angeordnet sind und
- das Befestigungsmittel (7) des Kieferverlagerungsmittels (1) auf dem Unterkiefer (MD) auf der Höhe eines der Backenzähne (21, 22) des unteren Zahnbogens (2) angeordnet ist, wenn die Vorrichtung (D) vom Benutzer getragen wird.

4. Vorrichtung (D) nach einem der vorhergehenden Ansprüche,
**dadurch gekennzeichnet, dass**
die Verbindungsmittel (14) ein Einstellungsmittel (16) hinsichtlich der Verlagerung des Unterkiefers (MD) relativ zum Oberkiefer (MX) aufweisen, das aus einem Gewinde (111) sowie einem mit einem Gewinde versehenen Röhrchen (121) besteht, wobei das Gewinde von dem oberen Bereich (11) und das Röhrchen (121) vom unteren Bereich (12) umfasst werden oder umgekehrt, wobei das Gewinde (111) in das mit Gewinde versehene Röhrchen (121) eingeschraubt wird.

5. Vorrichtung (D) nach einem der vorhergehenden Ansprüche,
**dadurch gekennzeichnet, dass**
die Vorrichtung wenigstens einen Aufbiss (4) umfasst, der dazu vorgesehen ist, am oberen oder unterem Zahnbogen (3, 2) direkt oder mittels eines Verbindungsmittels (61) befestigt zu werden.

6. Vorrichtung (D) nach einem der vorhergehenden Ansprüche,
**dadurch gekennzeichnet, dass**
sie weiterhin wenigstens einen Gummizug umfasst, der dazu vorgesehen ist, am Oberkiefer (MX) sowie am Unterkiefer (MD) mittels zweier Ankopplungsmittel befestigt zu werden, die jeweils direkt oder mittels Verbindungsmitteln (6, 7) an dem Oberkiefer (MX) und dem Unterkiefer (MD) befestigt sind.
